# EUROPEAN PATENT SPECIFICATION

(11) **EP 2 775 961 B1**
(45) Date of publication and mention of the grant of the patent: **26.12.2018**
(21) Application number: 12847265.1
(22) Date of filing: 08.11.2012
(51) Int. Cl.: A61F 2/16, A61F 2/14, A61F 2/04

(54) **ACCOMMODATING INTRAOCULAR LENSES**
AKKOMMODATIVE INTRAOKULARLINSE
LENTILLES INTRAOCULAIRES ADAPTATIVES

(30) Priority: 08.11.2011 US 201161557237 P
(43) Date of publication of application: 17.09.2014
(60) Divisional application: 18203988.3
(73) Proprietor: PowerVision, Inc., Belmont, CA 94002 (US)
(72) Inventor: SMILEY, Terah, Whiting, Belmont, CA 94002 (US); HILDEBRAND, Daniel, Belmont, CA 94002 (US); FLAHERTY, Bryan, Patrick, Belmont, CA 94002 (US)
(74) Representative: J A Kemp
(86) International application number: PCT/US2012/064166
(87) International publication number: WO 2013/070924

(56) References cited:
- WO-A1-2011/119334
- US-A1- 2004 169 816
- US-A1- 2004 169 816
- US-A1- 2007 213 817
- US-A1- 2008 306 588
- US-A1- 2009 005 865
- US-A1- 2009 248 154
- US-A1- 2010 179 653
- US-A1- 2010 179 653

## Description

### BACKGROUND

The crystalline lens is a transparent, biconvex structure in the eye that, along with the cornea, helps to refract light to be focused on the retina. The crystalline lens, by changing shape, functions to change the focal distance of the eye so that it can focus on objects at various distances. This adjustment of the crystalline lens is known as accommodation. The lens capsule is a smooth, transparent membrane that completely surrounds the lens. The lens capsule is elastic and is composed of collagen. The lens is flexible and its curvature is controlled by ciliary muscles through the zonules, which connect the ciliary muscles and the equatorial region of the capsule. At short focal distance the ciliary muscle contracts, the zonules loosen, and the lens thickens, resulting in a rounder shape and thus high refractive power. Changing focus to an object at a greater distance requires the relaxation of the ciliary muscle, which increases the tension on the zonules, flattening the lens and thus increasing the focal distance.

A crystalline lens can be removed and replaced with an artificial lens, generally referred to as an intraocular lens, for a variety of reasons. Some intraocular lenses are used to replace a cataract lens, a clouding that develops in the crystalline lens of the eye, obstructing the passage of light. Intraocular lenses can be characterized as non-accommodating or accommodating. Accommodating intraocular lenses are designed to function similarly to the native crystalline lens and are adapted to change power to provide near and distance vision.

The native crystalline lens is typically removed through a procedure referred to as an extracapsular extraction. The procedure includes making a capsulorhexis, a circular incision made on the anterior side of the capsule, followed by removal of the lens material. The replacement intraocular lens can then be positioned within the capsule through the opening formed by the circular incision.

US 2004/169816 A1 discloses an accommodating intraocular lens having optical parameters that are altered in-situ using forces applied by the ciliary muscles, in which a lens body carries an actuator separating two fluid-filled chambers having either the same index of diffraction or different indices of refraction, actuation of the actuator changing the relative volumes of fluid within an optic element of the lens and altering the optical power of the lens.

US 2009/005865 A1 discloses a n accommodating intraocular lens adapted for a post-implant modification, comprising: an optic portion; a non-optic portion disposed peripherally from the optic portion and adapted to engage a lens capsule, wherein the intraocular lens is adapted to change power in response to ciliary muscle movement; and an actuatable element adapted to be actuated by an external energy source to change an optical parameter of the intraocular lens. The optic portion and the non-optic portion are in fluidic communication, and the lens is adapted to move fluid between the optic portion and the non-optic portion in response to ciliary muscle movement to change the power of the lens. The actuatable element is disposed within the lens such that upon actuation the fluid is moved between the optic portion and the non-optic portion of the intraocular lens.

An accommodating intraocular lens according to the preamble of claim 1 is known from document US-A-2008/0306588.

### SUMMARY OF THE DISCLOSURE

According to one aspect of the present invention there is provided the accommodating intraocular lens of claim 1.

Additional aspects of the invention are set out in the dependent claims.

### BRIEF DESCRIPTION OF THE DRAWINGS

Figures 1A and 1B illustrate an exemplary accommodating intraocular lens.
Figure 1C illustrates a sectional view of the accommodating intraocular lens from Figures 1A and 1B.
Figure 1D is a top view of an exemplary posterior element of an accommodating intraocular lens.
Figure 1E is a sectional assembly view of an exemplary optic portion of an accommodating intraocular lens.
Figures 1F and 1G illustrate an exemplary haptic.
Figure 1H illustrate an exemplary coupling between an optic portion and a haptic.
Figures 2A-2C illustrate an exemplary haptic.
Figures 2D-2F illustrate sectional views of the haptic from Figure 2A.
Figure 2G illustrates an opening in a first end of the haptic from Figures 2A-2C.
Figure 3 illustrates exemplary diameters of an accommodating intraocular lens.
Figure 4 illustrates an exemplary haptic.
Figures 5A and 5B illustrate the deformation of an exemplary haptic in response to exemplary forces.
Figure 6 illustrates an exemplary fluid opening in an exemplary haptic.
Figure 7 illustrates an exemplary fluid opening in an exemplary haptic.
Figure 8 illustrates a sectional view of an exemplary accommodating intraocular lens.
Figure 9 illustrates a sectional view of an exemplary accommodating intraocular lens with relatively short haptics.

### DETAILED DESCRIPTION

The disclosure relates generally to accommodating intraocular lenses. In some embodiments the accommodating intraocular lenses described herein are adapted to be positioned within a native capsular bag in which a native lens has been removed. In these embodiments a peripheral non-optic portion (i.e., a portion not specifically adapted to focus light on the retina) is adapted to respond to capsular bag reshaping due to ciliary muscle relaxation and contraction. The response is a deformation of the peripheral portion that causes a fluid to be moved between the peripheral portion and an optic portion to change an optical parameter (e.g., power) of the intraocular lens.

The peripheral portions of the accommodating intraocular lenses described herein are adapted so that at least a portion of the peripheral portions is less responsive, or less sensitive, to certain types of capsular forces than to other types of capsular forces. Less responsive, or less-sensitive, as used herein, generally means that the optical power of the accommodating intraocular lens will change less in response to the types of forces to which the peripheral portion is less sensitive than to other types of forces. In general, the peripheral portions are adapted to be less responsive to forces in the anterior-to-posterior direction than to forces in the radial direction. In some cases the forces in the anterior-to-posterior direction are non-ciliary muscle related capsular forces, such as from size mismatch between the capsular bag and the intraocular lens, or from a capsular bag healing response. The radial forces as described herein are capsular reshaping and capsular forces resulting from ciliary muscle contraction and relaxation, causing accommodation of the accommodating intraocular lens. The accommodating intraocular lenses herein are thus considered to be more sensitive to radial forces than to forces in the anterior-to-posterior direction, and thus the optical power of the accommodating intraocular lens will change more in response to the radial forces than it will in response to forces in the anterior-to-posterior direction.

One of the benefits of the peripheral portions described herein is that they reshape the capsule, by essentially "propping" it open, in a predictable way while still preserving the radial sensitivity of the peripheral portion to radial forces to allow the accommodating lens to accommodate. Variations in the base state of the accommodating intraocular lens due to one or more of anatomical variations in capsule size, inaccurate capsule measurements, or post-implant changes in the capsule are reduced because the peripheral portion is adapted to more predictably reshape the capsule in at least one direction. In some embodiments the peripheral portion is adapted to reshape the capsule in a more predictable way because it is stiffer in at least one direction. For example, in some embodiments the peripheral portion is stiffer in the anterior-to-posterior direction than in the radial direction. In these embodiments the peripheral portion is adapted to prop open the capsule in the anterior-to-posterior direction.

As used herein, "anterior-to-posterior," or derivatives thereof, is not intended to be limited to the direction that is perfectly parallel to the optical axis, but is interpreted to mean a direction that is generally in what is typically referred to as the anterior-to-posterior direction. For example without limitation, the "anterior-to-posterior" direction includes directions or axes that are 10 degrees from the optical axis of the accommodating intraocular lens. The "radial" forces described herein are not to be considered to be in the anterior-to-posterior direction.

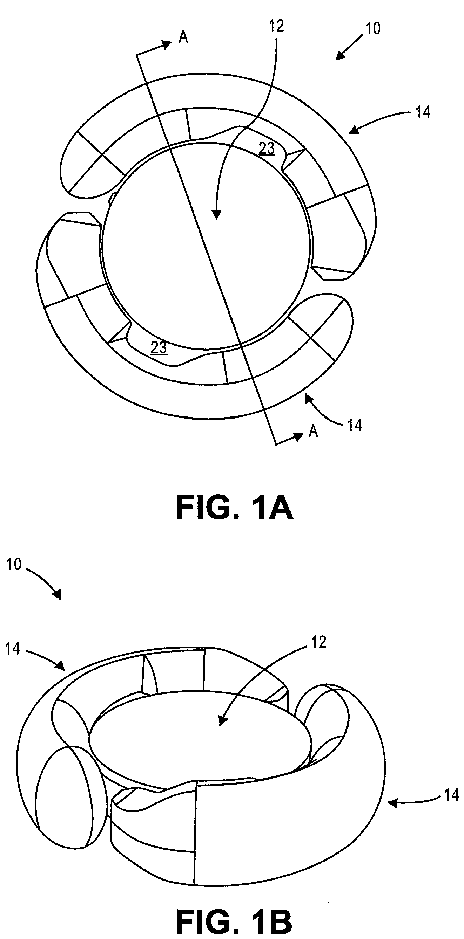

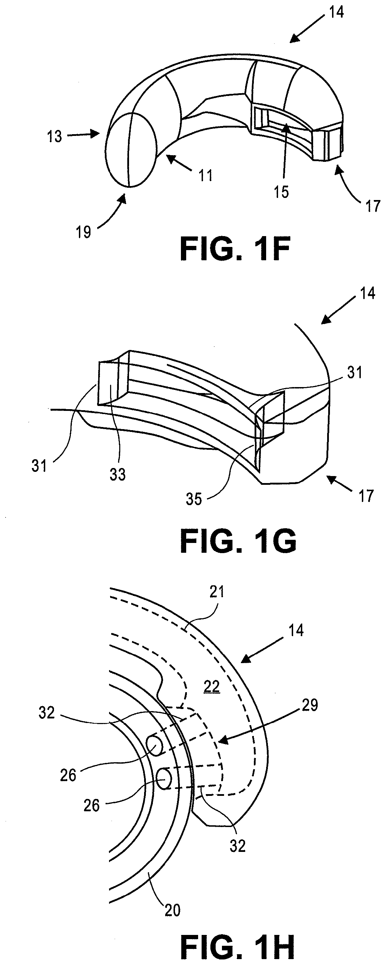

Figure 1A is a top view illustrating accommodating intraocular lens 10 that includes optic portion 12 and a peripheral portion that in this embodiment includes first and second haptics 14 coupled to and extending peripherally from optic portion 12. Optic portion 12 is adapted to refract light that enters the eye onto the retina. Haptics 14 are configured to engage a capsular bag and are adapted to deform in response to ciliary muscle related capsular bag reshaping. Figure 1B is a perspective view of intraocular lens 10 showing optic portion 12 and haptics 14 coupled to optic portion 12.

The haptics are in fluid communication with the optic portion. Each haptic has a fluid chamber that is in fluid communication with an optic chamber in the optic portion. The haptics are formed of a deformable material and are adapted to engage the capsular bag and deform in response to ciliary muscle related capsular bag reshaping. When the haptics deform the volume of the haptic fluid chamber changes, causing a fluid disposed in the haptic fluid chambers and the optic fluid chamber to either move into the optic fluid chamber from the haptic fluid chambers, or into the haptic fluid chambers from the optic fluid chamber. When the volume of the haptic fluid chambers decreases, the fluid is moved into the optic fluid chamber. When the volume of the haptic fluid chamber increases, fluid is moved into the haptic fluid chambers from the optic fluid chamber. The fluid flow into and out of the optic fluid chamber changes the configuration of the optic portion and the power of the intraocular lens.

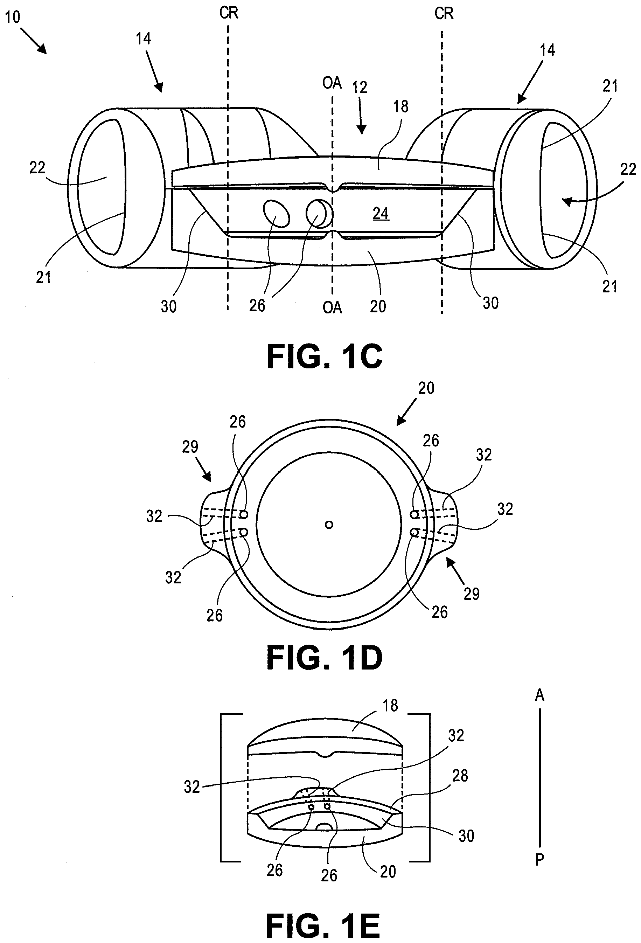

Figure 1C is a side sectional view through Section A-A indicated in Figure 1A. Optic portion 12 includes deformable anterior element 18 secured to deformable posterior element 20. Each haptic 14 includes a fluid chamber 22 that is in fluid communication with optic fluid chamber 24 in optic portion 12. Only the coupling between the haptic 14 to the left in the figure and option portion 12 is shown (although obscured) in the sectional view of Figure 1C. The haptic fluid chamber 22 to the left in the figure is shown in fluid communication with optic fluid chamber 24 via two apertures 26, which are formed in posterior element 20. The haptic 14 to the right in Figure 1C is in fluid communication with optic chamber 24 via two additional apertures also formed in posterior element (not shown) substantially 180 degrees from the apertures shown.

Figure 1D is a top view of posterior element 20 (anterior element 18 and haptics 14 not shown). Posterior element 20 includes buttress portions 29 in which channels 32 are formed. Channels 32 provide fluid communication between optic portion 12 and haptics 14. Apertures 26 are disposed at one end of channels 32. The optic fluid chamber 24 is therefore in fluid communication with a single haptic via two fluid channels. Buttress portions 29 are configured and sized to be disposed within an opening formed in haptics 14 that defines one end of the haptic fluid chamber, as described below. Each of buttress portions 29 includes two channels formed therein. A first channel in a first buttress is in alignment with a first channel in the second buttress. The second channel in the first buttress is in alignment with the second channel in the second buttress.

There are advantages to having two channels in each buttress as opposed to one channel. A design with two channels rather than one channel helps maintain dimensional stability during assembly, which can be important when assembling flexible and thin components. Additionally, it was observed through experimentation that some one-channel designs did not provide adequate optical quality throughout the range of accommodation. In particular, lens astigmatism was observed in some one-channel designs, particularly as the intraocular lens accommodated. It was discovered that the two-channel buttress designs described herein reduced astigmatism, particularly as the lens accommodated. Astigmatism is reduced in these embodiments because the stiffness of the buttress is increased by the rib portion between the two channels. The additional stiffness results in less deflection due to pressure changes in the channels. Less deflection due to the pressure changes in the channels results in less astigmatism. In some embodiments the channels are between about .4 mm and about .6 mm in diameter. In some embodiments the channels are about .5 mm in diameter. In some embodiments the distance between the apertures is about .1 mm to about 1.0 mm.

Figure 1E is a side assembly view through section A-A of optic portion 12, which includes anterior element 18 and posterior element 20 (haptics not shown for clarity). By including fluid channels 32 in posterior element 20, posterior element 20 needs to have enough structure through which the channels 32 can be formed. Buttress portions 29 provide that structures in which channels 32 can be formed. At its peripheral-most portion posterior element 20 is taller than anterior element 18 in the anterior-to-posterior direction. In alternative embodiments, the channels can be formed in anterior element 18 rather than posterior element 20. The anterior element would include buttress portions 29 or other similar structure to provide structure in which the channels can be formed. In these alternative embodiments the posterior element could be formed similarly to anterior element 18.

As shown in Figure IE, posterior element 20 is secured to anterior element 18 at peripheral surface 28, which extends around the periphery of posterior element 20 and is a flat surface. Elements 18 and 20 can be secured together using known biocompatible adhesives. Anterior element 18 and posterior element 20 can also be formed from one material to eliminate the need to secure two elements together. In some embodiments the diameter of the region at which anterior element 18 and posterior element 20 are secured to one another is about 5.4 mm to about 6 mm in diameter.

In some embodiments the thickness of anterior element 18 (measured in the anterior-to-posterior direction) is greater along the optical axis ("OA" in Figure 1C) than at the periphery. In some embodiments the thickness increases continuously from the periphery towards the thickest portion along the optical axis.

In some embodiments the thickness of posterior element 20 decreases from the location along the optical axis towards the edge of central region "CR" identified in Figure 1C. The thickness increases again radially outward of central region CR towards the periphery, as can be seen in Figure 1C. In some particular embodiments central region CR is about 3.75 mm in diameter. The apertures are formed in beveled surface 30.

In some embodiments the thickness of posterior element 20 along the optical axis is between about 0.45 mm and about 0.55 mm and the thickness at the periphery of posterior element 20 is between about 1.0 mm and about 1.3.

In some embodiments the thickness of posterior element 20 along the optical axis is about 0.5 mm and the thickness at the periphery of posterior element 20 is about 1.14 mm.

In some embodiments the thickness of anterior element 18 along the optical axis is between about 0.45 mm to about .55 mm, and in some embodiments is between about 0.50 mm to about 0.52 mm. In some embodiments the thickness at the periphery of anterior element 18 is between about 0.15 mm and about 0.4 mm, and in some embodiments is between about 0.19 mm and about 0.38 mm.

In one particular embodiment the thickness of anterior element 18 along the optical axis is about 0.52 mm and the thickness of the periphery of anterior element 18 is about 0.38 mm, and the thickness of posterior element 20 along the optical axis is about 0.5 mm and the thickness at the periphery of posterior element 20 is about 1.14 mm.

In one particular embodiment the thickness of anterior element 18 along the optical axis is about 0.5 mm and the thickness of the periphery of anterior element 18 is about 0.3 mm, and the thickness of posterior element 20 along the optical axis is about 0.5 mm and the thickness at the periphery of posterior element 20 is about 1.14 mm.

In one particular embodiment the thickness of anterior element 18 along the optical axis is about 0.51 mm and the thickness of the periphery of anterior element 18 is about 0.24 mm, and the thickness of posterior element 20 along the optical axis is about 0.5 mm and the thickness at the periphery of posterior element 20 is about 1.14 mm.

In one particular embodiment the thickness of anterior element 18 along the optical axis is about 0.52 mm and the thickness of the periphery of anterior element 18 is about 0.19 mm, and the thickness of posterior element 20 along the optical axis is about 0.5 mm and the thickness at the periphery of posterior element 20 is about 1.14 mm.

The optic portion is adapted to maintain optical quality throughout accommodation. This ensures that as the accommodating intraocular lens transitions between the disaccommodated and accommodated configurations, the optic portion maintains optical quality. A number of factors contribute to this beneficial feature of the accommodating intraocular lenses herein. These factors include the peripheral region at which anterior element 18 is secured to posterior element 20, the shape profile of the anterior element 18 and posterior element 20 inside central region CR of the optic portion (see Figure 1C), and the thickness profiles of anterior element 18 and posterior element 20. These contributing factors ensure that both the anterior and posterior elements flex in such a way as to maintain the shape necessary to maintain optical quality across a range of optical powers.

Figure IF illustrates one haptic 14 from intraocular lens 10 (optic portion 12 and the second haptic not shown for clarity). Haptic 14 includes radially outer portion 13 adapted to face the direction of the zonules, and radially inner portion 11, which faces the periphery of the optic (not shown). Haptic 14 includes a first end region 17 which is secured to optic portion 12, and second end region 19 that is closed. Haptic 14 also includes opening 15 in first end region 17 that provides the fluid communication with the haptic. In this embodiment opening 15 is sized and configured to receive buttress portion 29 of optic portion 12 therein.

Figure 1G is a close up view of opening 15 in haptic 14, which is adapted to receive buttress portion 29 therein. The opening 15 has curved surfaces 33 and 35 that are shaped to mate with curved surfaces on the optic buttress 29. Surface 31 surrounds opening 15 and provides a surface to which a corresponding surface of the optic can be secured.

Figure 1H is a top close up view of buttress portion 29 (in phantom) from posterior element 20 disposed within opening 15 in haptic 14 (anterior element of the optic not shown for clarity). Channels 32 are shown in phantom. Haptic 14 includes fluid chamber 22 defined by inner surface 21. Fluid moves between the optic fluid chamber and haptic fluid chamber 22 through channels 32 upon the deformation of haptic 14.

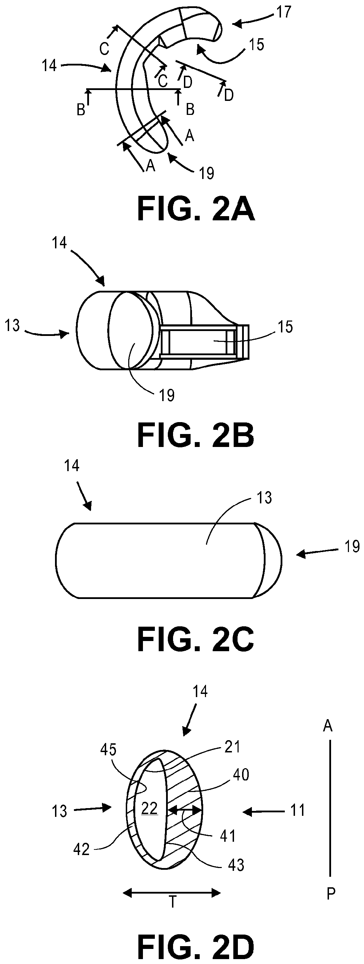

Figure 2A is a top view showing one haptic 14 shown in Figures 1A-1H. The optic portion and the second haptic are not shown. Four sections A-D are identified through the haptic. Figure 2B illustrates a side view of haptic 14, showing opening 15 and closed end 19. Figure 2C is a side view of haptic 14 showing radially outer portion 13 and closed end 19.

Figure 2D is the cross sectional view through section A-A shown in Figure 2A. Of the four sections shown in Figure 2A, section A-A is the section closest to closed end 19. Radially inner portion 11 and radially outer portion 13 are identified. Fluid channel 22 defined by surface 21 is also shown. In this section the radially inner portion 40 is radially thicker (in the direction "T") than radially outer portion 42. Inner portion 40 provides the haptic's stiffness in the anterior-to-posterior direction that more predictably reshapes the capsule in the anterior-to-posterior direction. Radially inner portion 40 has a greatest thickness dimension 41, which is along an axis of symmetry in this cross section. The outer surface of haptic 14 has a generally elliptical configuration in which the greatest height dimension, in the anterior-to-posterior direction ("A-P"), is greater than the greatest thickness dimension (measured in the "T" dimension). The fluid chamber 22 has a general D- shaped configuration, in which the radially inner wall 43 is less curved (but not perfectly linear) than radial outer wall 45. Radially outer portion 42 engages the capsular bag where the zonules attach thereto, whereas the thicker radially portion 40 is disposed adjacent the optic.

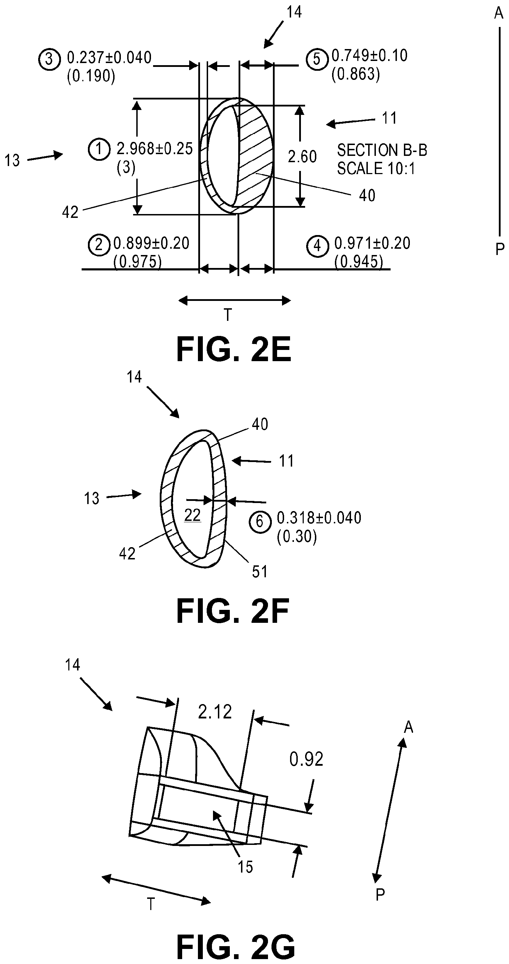

Figure 2E illustrates section B-B shown in Figure 2A. Section B-B is substantially the same as section A-A, and Figure 2E provides exemplary dimensions for both sections. Radially inner portion 40 has a greatest thickness along the midline of about .75 mm (in the radial direction "T"). Radially outer portion 42 has a thickness along the midline of about .24 mm. Fluid chamber 22 has a thickness of about .88 mm. Haptic 14 has a thickness along the midline of about 1.87 mm. The height of the haptic in the anterior to posterior dimension is about 2.97 mm. The height of the fluid chamber is about 2.60 mm. In this embodiment the thickness of the radially inner portion 40 is about 3 times the thickness of the radially outer portion 42. In some embodiments the thickness of the radially inner portion 40 is about 2 times the thickness of the radially outer portion 42. In some embodiments the thickness of the radially inner portion 40 is about 2 to about 3 times the thickness of the radially outer portion 42. In some embodiments the thickness of the radially inner portion 40 is about 1 to about 2 times the thickness of the radially outer portion 42.

Fluid chamber 22 is disposed in the radially outer portion of haptic 14. Substantially the entire radially inner region of haptic 14 in this section is bulk material. Since the fluid chamber 22 is defined by surfaces 43 and 45 (see Figure 2D), the positioning and size of fluid chamber 22 depends on the thickness of the radially inner portion 40 and the radially outer portion 42.

Figure 2F illustrates Section C-C shown in Figure 2A. In Section C-C radially inner portion 40 is not as thick as radially inner portion 40 in sections A-A and B-B, although in Section C-C radially inner portion 40 is slightly thicker than radially outer portion 42. In this particular embodiment radially inner portion 40 is about .32 mm in Section C-C. Radially outer portion 42 has a thickness about the same as the radially outer thickness in Sections A-A and B-B, about .24 mm. The outer surface of haptic 14 does not have the same configuration as the outer surface in Sections A-A and Section B-B. In Section C-C the radially inner outer surface of haptic 51 is more linear than in Sections A-A and Section B-B, giving the outer surface of haptic in Section C-C a general D-shape. In Section C-C fluid chamber 22 has a general D-shape, as in Sections A-A and Section B-B. The haptic, in Section C-C has a fluid chamber configuration that is substantially the same as the fluid chamber configurations in Sections A-A and B-B, but has an outer surface with a configuration different than the configuration of the outer surface of haptic 14 in Sections A-A and B-B.

The thinner radially inner portion 40 in Section C-C also creates access pathways 23 that are shown in Figure 1A. This space between optic portion 12 and haptics 14 allows a physician to insert one or more irrigation and/or aspiration devices into space 23 during the procedure and apply suction to remove viscoelastic fluid that may be used in the delivery of the intraocular lens into the eye. The pathways 23 could also be anywhere along the length of the haptic, and there could be more than one pathway 23. In this connection reference is made to the disclosure in Figures 23 and 24, and the textual description thereof, in U.S. Pub. No. 2008/0306588, which include a plurality of pathways in the haptics.

Figure 2G shows a view through Section D-D from Figure 2A. Haptic 14 includes opening 15 therein, which is adapted to receive the buttress from the optic portion as described herein. The height of opening 15 in this embodiment is about .92 mm. The width, or thickness, of the opening is about 2.12 mm.

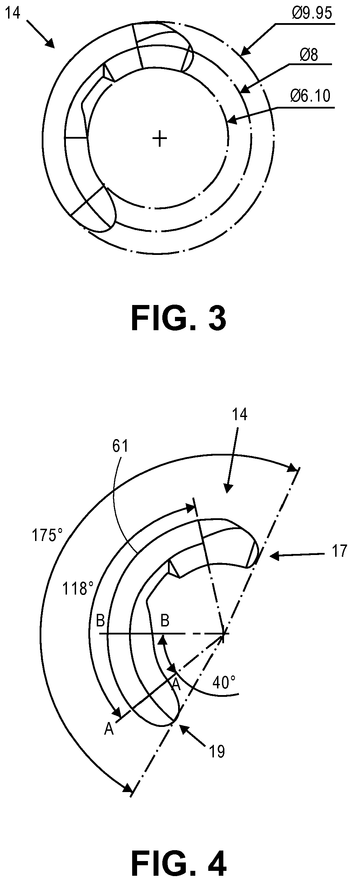

Figure 3 illustrates relative diameters of optic portion 12 (not shown) and of the peripheral portion, which includes two haptics 14 (only one haptic is shown). In this embodiment the optic has a diameter of about 6.1 cm, while the entire accommodating intraocular lens, including the peripheral portion, has a diameter of about 9.95 cm. The dimensions provided are not intended to be strictly limiting.

Figure 4 is a top view of haptic 14, showing that haptic 14 subtends an angle of about 175 degrees around optic (i.e., substantially 180 degrees). The optic portion is not shown for clarity. The two haptics therefore each subtend an angle of about 180 degrees around the optic. A first region 61 of haptic 14 is shown to subtend exemplary angle of about 118 degrees. This is the radially outermost portion of haptic 14, is adapted to engage the capsular bag, and is adapted to be most responsive to capsular shape changes. Region 61 can be thought of as the most responsive part of haptic 14.

The angle between Sections A-A and B-B, which are considered the boundaries of the stiffer radially inner portion of the haptic, is about 40 degrees. The stiff radially inner portion of haptic 14 is positioned directly adjacent the periphery of the optic. The dimensions and angles provided are not intended to be strictly limiting.

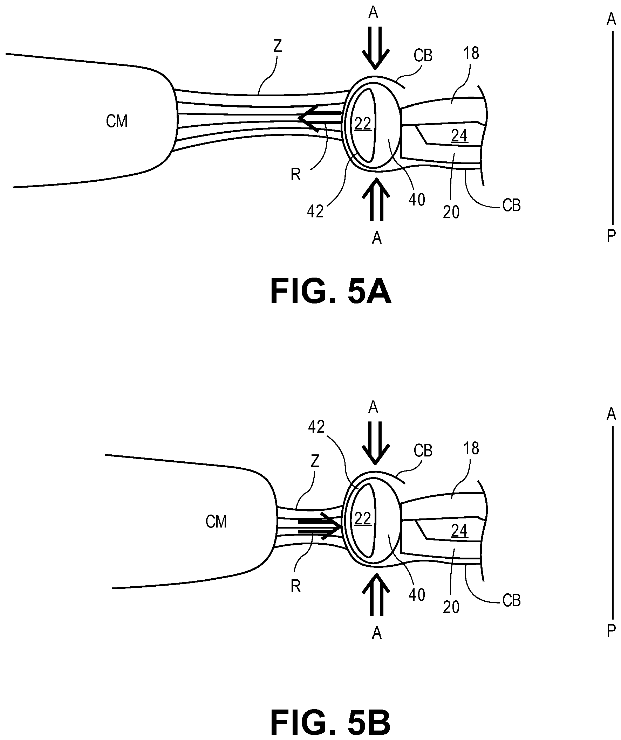

Figures 5A and 5B illustrate a portion of accommodating intraocular lens 10 positioned in a capsular bag ("CB") after a native lens has been removed from the CB. The anterior direction is on top and the posterior direction is on bottom in each figure. Figure 5A shows the accommodating intraocular lens in a lower power, or dis-accommodated, configuration relative to the high power, or accommodated, configuration shown in Figure 5B.

The elastic capsular bag "CB" is connected to zonules "Z," which are connected to ciliary muscles "CM." When the ciliary muscles relax, as shown in Figure 5A, the zonules are stretched. This stretching pulls the capsular bag in the generally radially outward direction due to radially outward forces "R" due to the general equatorial connection location between the capsular bag and the zonules. The zonular stretching causes a general elongation and thinning of the capsular bag. When the native lens is still present in the capsular bag, the native lens becomes flatter (in the anterior-to-posterior direction) and taller in the radial direction, which gives the lens less power. Relaxation of the ciliary muscle, as shown in Figure 5A, provides for distance vision. When the ciliary muscles contract, however, as occurs when the eye is attempting to focus on near objects, the radially inner portion of the muscles move radially inward, causing the zonules to slacken. This is illustrated in Figure 5B. The slack in the zonules allows the capsular bag to move towards a generally more curved configuration in which the anterior surface has greater curvature than in the disaccommodated configuration, providing higher power and allowing the eye to focus on near objects. This is generally referred to as "accommodation," and the lens is said to be in an "accommodated" configuration.

In section A-A (which is the same as section B-B) of haptic 14, illustrated in Figures 5A and 5B, radially inner portion 40 includes thicker bulk material that provides haptic 14 with stiffness in the anterior-to-posterior direction. When capsular bag forces are applied to the haptic in the anterior-to-posterior direction, the inner portion 40, due to its stiffness, deforms in a more repeatable and predictable manner making the base state of the lens more predictable. Additionally, the haptic, due to its stiffer inner portion, deforms the capsule in a repeatable way in the anterior-to-posterior direction. Additionally, because the haptic is less flexible along the length of the haptic, the accommodating intraocular lens's base state is more predictable because bending along the length of the haptic is one way in which fluid can be moved into the optic (and thereby changing the power of the lens). Additional advantages realized with the stiffer inner portion are that the haptics are stiffer to other forces such as torqueing and splaying because of the extra bulk in the inner portion.

The radially outer portion 42 is the portion of the haptic that directly engages the portion of the capsular bag that is connected to the zonules. Outer portion 42 of the haptics is adapted to respond to capsular reshaping forces "R" that are applied generally radially when the zonules relax and stretch. This allows the haptic to deform in response to ciliary muscle related forces (i.e., capsular contraction and relaxation) so that fluid will flow between the haptic and the optic in response to ciliary muscle relaxation and contraction. This is illustrated in Figure 5B. When the ciliary muscles contract (Figure 5B), the peripheral region of the elastic capsular bag reshapes and applies radially inward forces "R" on radially outer portion 42 of haptic 14. The radially outer portion 42 is adapted to deform in response to this capsular reshaping. The deformation decreases the volume of fluid channel 22, which forces fluid from haptic chamber 22 into optic chamber 24. This increases the fluid pressure in optic chamber 42. The increase in fluid pressure causes flexible anterior element 18 and flexible posterior element 20 to deform, increasing in curvature, and thus increasing the power of the intraocular lens.

The haptic is adapted to be stiffer in the anterior-to-posterior direction than in the radial direction. In this embodiment the radially outer portion 42 of haptic 14 is more flexible (i.e., less stiff) in the radial direction than the stiffer inner portion 40 is in the anterior-to-posterior direction. This is due to the relative thicknesses of outer portion 42 and inner portion 40. The haptic is thus adapted to deform less in response to forces in the anterior-to-posterior direction than to forces in the radial direction. This also causes less fluid to be moved from the haptic into the optic in response to forces in the anterior-to-posterior direction than is moved into the optic in response to forces in the radial direction. The haptic will also deform in a more predictable and repeatable manner due to its stiffer radially inner portion.

The peripheral portion is thus more sensitive to capsular bag reshaping in the radial direction than to capsular bag reshaping in the anterior-to-posterior direction. The haptics are adapted to deform to a greater extent radially than they are in the anterior-to-posterior direction. The disclosure herein therefore includes a peripheral portion that is less sensitive to capsular forces along a first axis, but is more sensitive to forces along a second axis. In the example above, the peripheral portion is less sensitive along the posterior-to-anterior axis, and is more sensitive in the radial axis.

An exemplary benefit of the peripheral portions described above is that they deform the capsular bag in a repeatable way and yet maintain a high degree of sensitivity to radial forces during accommodation. The peripheral portions described above are stiffer in the anterior-to-posterior direction than in the radial direction.

An additional example of capsular forces in the anterior-to-posterior direction is capsular forces on the peripheral portion after the accommodating intraocular lens is positioned in the capsular bag, and after the capsular bag generally undergoes a healing response. The healing response generally causes contraction forces on the haptic in the anterior-to-posterior direction, identified in Figure 5A by forces "A." These and other post-implant, such as non-accommodating-related, capsular bag reshaping forces are described in US 2010/179653 A1. For example, there is some patient to patient variation in capsular bag size, as is also described in detail in US 2010/179653 A1. When an intraocular lens is positioned within a capsular bag, size differences between the capsule and intraocular lens may cause forces to be exerted on one or more portions of the intraocular lens in the anterior-to-posterior direction.

In the example of capsular healing forces in the anterior-to-posterior direction, the forces may be able to deform a deformable haptic before any accommodation occurs. This deformation changes the volume of the haptic fluid chamber, causing fluid to flow between the optic fluid chamber and the haptic fluid chambers. This can, in some instances undesirably, shift the base power of the lens. For example, fluid can be forced into the optic upon capsular healing, increasing the power of the accommodating intraocular lens, and creating a permanent myopic shift for the accommodating intraocular lens. Fluid could also be forced out of the optic and into the haptics, decreasing the power of the accommodating intraocular lens.

As used herein, "radial" need not be limited to exactly orthogonal to the anterior-to-posterior plane, but includes planes that are 45 degrees from the anterior-to-posterior plane.

Exemplary fluids are described in US 2010/179653 A1 and in US 2011/208301 A1. For example, the fluid can be a silicone oil that is or is not index-matched with the polymeric materials of the anterior and posterior elements. When using a fluid that is index matched with the bulk material of the optic portion, the entire optic portion acts a single lens whose outer curvature changes with increases and decreases in fluid pressure in the optic portion.

In the embodiment in Figures 2A-2G above the haptic is a deformable polymeric material that has a substantially uniform composition in Sections A-A, B-B, and C-C. The stiffer radially inner body portion 40 is attributed to its thickness. In alternative embodiments the radially inner body portion has a different composition that the outer body portion, wherein the radially inner body portion material is stiffer than the material of the radially outer body portion. In these alternative embodiments the thicknesses of the radially inner and outer portions can be the same.

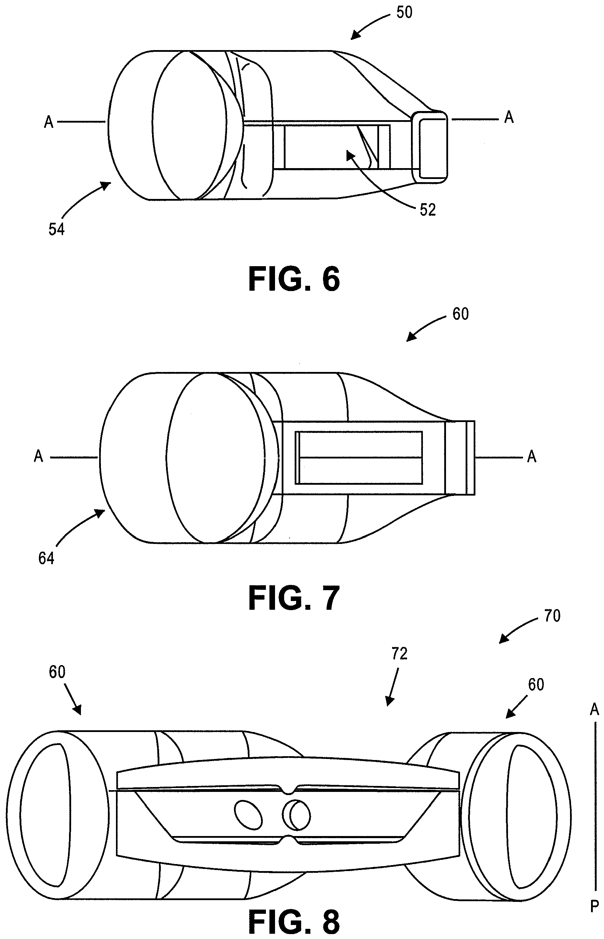

Figure 6 illustrates haptic 50, which is the same haptic configuration as in shown in Figure 2B. The radially outer portion 54 is identified. The haptic has axis A-A halfway through the height of the haptic. Opening 52, in which the optic buttress is disposed, is on the posterior side of axis A. In this embodiment the optic sits slightly closer to the posterior-most portion of the haptics than the anterior-most portion of the haptics.

Figure 7 illustrates an alternative haptic 60 (optic not shown), wherein the radially outer portion 64 is identified. Haptic 60 includes axis A-A halfway through the thickness of the haptic. Opening 62 is symmetrical about the axis A. Additionally, axis A-A is an axis of symmetry for haptic 60. The symmetry of the haptic along axis A can improve the ability to mold low relatively low stress components. Figure 8 shows an embodiment of intraocular lens 70 in which the optic 72 is coupled to two haptics 60, which are the haptics shown in Figure 7. The optic sits further in the anterior direction that in the embodiment in which the opening is not along the midline of the haptic. The cross sections A-A, B-B, and C-C of haptic 60 are the same as those shown in other embodiments shown above.

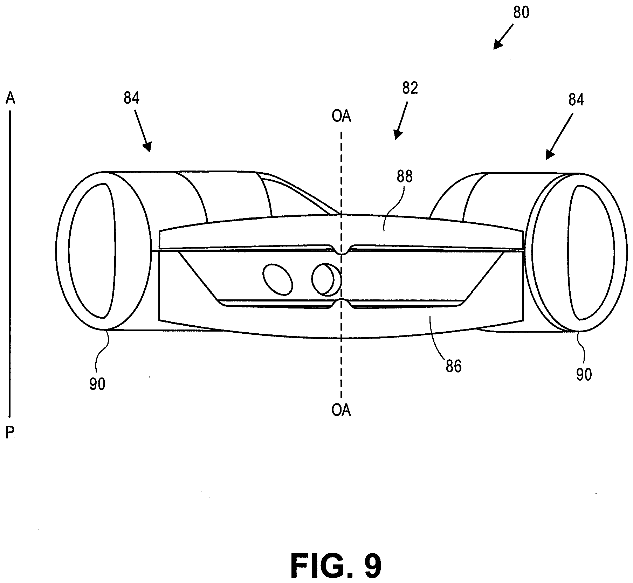

Figure 9 illustrates intraocular lens 80 including optic 82 and two haptics 84. The optic is the same as the optic portions described herein. Haptics 84 are not as tall, measured in the anterior-to-posterior direction, as haptic 60, haptic 50, or haptic 14. In exemplary embodiments haptics 84 are between about 2.0 mm and about 3.5 mm tall, and in some embodiments they are about 2.8 mm tall. Intraocular lens 80 can be considered a size "small" accommodating intraocular lens for patients with a capsular bag that is below a certain threshold size. The posterior surface of posterior element 86 is disposed slightly further in the posterior direction than the posterior-most portions 90 of haptics 84.

Characteristics of the intraocular lenses described herein can similarly be applied to non-fluid driven accommodating intraocular lenses. For example, a non-accommodating intraocular lens can include a peripheral portion with a first stiffer region that provides a region of the peripheral portion with an insensitivity in a first direction. For example, in an intraocular lens with two lenses adapted to be moved apart from one another to change the power of the lens, the peripheral portion of the lens can be adapted such that a first type of capsular reshaping does not cause the distance between the lenses to change, and thus the power of the intraocular lens stays the same.

Additionally, the accommodating intraocular lenses herein can also be adapted to be positioned outside of a native capsular bag. For example, the accommodating intraocular lenses can be adapted to be positioned in front of, or anterior to, the capsular bag after the native lens has been removed or while the native lens is still in the capsular bag, wherein the peripheral portion of the lens is adapted to respond directly with ciliary muscle rather than rely on capsular reshaping.

## Claims

1. An accommodating intraocular lens, comprising:
an optic portion (12) comprising an optic fluid chamber (24) and an optical axis (OA); and
a haptic (14) with a haptic fluid chamber (22) in fluid communication with the optic fluid chamber (24), the haptic (14) having a proximal end (17) coupled to the optic potion (12) and a closed distal end (19) away from the proximal end (17), wherein the haptic (14) is adapted to engage a capsular bag and deformable to move the fluid between the haptic fluid chamber (22) and the optic fluid chamber (24) to change an optical parameter of the accommodating intraocular lens,
wherein, in a region of the haptic (14) that is adaptd to engage the capsular bag, the haptic (14) has a first cross section (A-A) in a plane that extends in the anterior-to-posterior direction and includes the optical axis (OA) in which an outer surface of the haptic (14) has a first configuration, and a second cross section (C-C) in a plane that extends in the anterior-to-posterior direction and includes the optical axis (OA) in which an outer surface of the haptic (14) has a second configuration different than the first configuration, wherein the second cross section (C-C) is between the first cross section (A-A) and the proximal end (17) of the haptic (14), at the second cross section (C-C) the haptic (14) is spaced apart from the optic portion (12) more than at the first cross section (A-A) to provide an access pathway (23) at the second cross section (C-C), **characterised in that**
in the first cross section (A-A) the haptic (14) has a radially inner portion (40) with a first thickness (41) measured in a radial direction relative to the optical axis (OA), and wherein in the second cross section (C-C) the haptic (14) has a radially inner portion (40) with a second thickness measured in a radial direction relative to the optical axis (OA), the second thickness less than the first thickness, and
wherein at the first cross section (A-A) the haptic (14) is directly adjacent the periphery of the optic portion (12),.

2. The accommodating intraocular lens of claim 1 wherein the first cross section (A-A) has an outer surface with a generally oval configuration.

3. The accommodating intraocular lens of claim 1 wherein the second cross section (C-C) has an outer surface with a general D-shaped configuration.

4. The accommodating intraocular lens of claim 1 wherein in the first cross section (A-A) the haptic fluid chamber (22) has a first fluid chamber configuration, and in the second cross section (C-C) the haptic fluid chamber (22) has a second fluid chamber configuration that is substantially the same as the first fluid chamber configuration.

5. The accommodating intraocular lens of claim 4 wherein the first fluid chamber configuration and the second fluid chamber configuration have a radially inner surface (43) that is more linear than a radially outer surface.

6. The accommodating intraocular lens of claim 4 wherein the first fluid chamber configuration and the second fluid chamber configuration are substantially D-shaped.

7. The accommodating intraocular lens of claim 1 wherein in the first cross (A-A) the radially inner portion (40) is at least twice as thick as a radially outer portion (42).

## Patentansprüche

1. Akkommodierende Intraokularlinse, umfassend:
ein optisches Teil (12), umfassend eine optische Fluidkammer (24) und eine optische Achse (OA); und
eine Haptik (14) mit einer haptischen Fluidkammer (22) in Fluidverbindung mit der optischen Fluidkammer (24), wobei die Haptik (14) ein proximales Ende (17), das an das optische Teil (12) gekoppelt ist, und ein geschlossenes distales Ende (19), das vom proximalen Ende (17) entfernt ist, hat, wobei die Haptik (14) dazu angepasst ist, mit einem Kapselsack im Eingriff zu sein, und verformbar ist, um das Fluid zwischen der haptischen Fluidkammer (22) und der optischen Fluidkammer (24) zu bewegen, um einen optischen Parameter der akkommodierenden Intraokularlinse zu verändern,
wobei in einem Bereich der Haptik (14), der angepasst ist, im Eingriff zu sein mit dem Kapselsack, die Haptik (14) einen ersten Querschnitt (A-A) in einer Ebene, der sich in anteriorposteriore Richtung erstreckt und die optische Achse (OA) umfasst, in der eine Außenfläche der Haptik (14) eine erste Konfiguration hat, und einen zweiten Querschnitt (C-C) in einer Ebene, der sich in anteriorposteriore Richtung erstreckt und die optische Achse (OA) umfasst, in der eine Außenfläche der Haptik (14) eine zweite Konfiguration hat, die sich von der ersten unterscheidet, hat, wobei der zweite Querschnitt (C-C) zwischen dem ersten Querschnitt (A-A) und dem proximalen Ende (17) der Haptik (14) ist, am zweiten Querschnitt (C-C) die Haptik (14) weiter vom optischen Teil (12) beabstandet ist als beim ersten Querschnitt (A-A), um einen Zugangsweg (23) am zweiten Querschnitt (C-C) bereitzustellen, **dadurch gekennzeichnet, dass**
im ersten Querschnitt (A-A) die Haptik (14) ein radial inneres Teil (40) mit einer ersten Dicke (41) hat, die in eine radiale Richtung bezüglich zur optischen Achse (OA) gemessen wird, und wobei im zweiten Querschnitt (C-C) die Haptik (14) ein radial inneres Teil (40) mit einer zweiten Dicke hat, die in eine radiale Richtung bezüglich der optischen Achse (OA) gemessen wird, die zweite Dicke geringer als die erste Dicke, und
wobei beim ersten Querschnitt (A-A) die Haptik (14) unmittelbar neben dem Rand des optischen Teils (12) ist.

2. Akkommodierende Intraokularlinse nach Anspruch 1, wobei der erste Querschnitt (A-A) eine Außenfläche mit einer im Allgemeinen ovalen Konfiguration hat.

3. Akkommodierende Intraokularlinse nach Anspruch 1, wobei der zweite Querschnitt (C-C) eine Außenfläche mit einer im Allgemeinen D-förmigen Konfiguration hat.

4. Akkommodierende Intraokularlinse nach Anspruch 1, wobei im ersten Querschnitt (A-A) die haptische Fluidkammer (22) eine erste Fluidkammerkonfiguration hat und im zweiten Querschnitt (C-C) die haptische Fluidkammer (22) eine zweite Fluidkammerkonfiguration hat, die im Wesentlichen dieselbe ist wie die erste Fluidkammerkonfiguration.

5. Akkommodierende Intraokularlinse nach Anspruch 4, wobei die erste Fluidkammerkonfiguration und die zweite Fluidkammerkonfiguration eine radiale Innenfläche (43) haben, die linearer ist als eine radiale Außenfläche.

6. Akkommodierende Intraokularlinse nach Anspruch 4, wobei die erste Fluidkammerkonfiguration und die zweite Fluidkammerkonfiguration im Wesentlichen D-förmig sind.

7. Akkommodierende Intraokularlinse nach Anspruch 1, wobei im ersten Quer (A-A) das radial äußere Teil (40) mindestens doppelt so dick ist wie ein radial inneres Teil (42).

## Revendications

1. Lentille intraoculaire accommodative, comprenant :
une partie optique (12) comprenant une chambre de fluide optique (24) et un axe optique (OA) ; et
une haptique (14) avec une chambre de fluide haptique (22) en communication fluidique avec la chambre de fluide optique (24), l'haptique (14) ayant une extrémité proximale (17) couplée à la partie optique (12) et une extrémité distale fermée (19) en éloignement de l'extrémité proximale (17), l'haptique (14) étant adaptée pour mettre en prise un sac capsulaire et déformable pour déplacer 1 fluide entre la chambre de fluide haptique (22) et la chambre de fluide optique (24) pour modifier un paramètre optique de la lentille intraoculaire accommodative,
dans laquelle, dans une région de l'haptique (14) qui est adaptée pour mettre en prise le sac capsulaire, l'haptique (14) présente une première section transversale (A-A) dans un plan qui s'étend dans la direction antérieure-postérieure et comprend l'axe optique (OA) dans lequel la surface externe de l'haptique (14) présente une première configuration, et une deuxième section transversale (C-C) dans un plan qui s'étend dans la direction antérieure-postérieure et comprend l'axe optique (OA) dans lequel la surface externe de l'haptique (14) présente une deuxième configuration différente de la première configuration, la deuxième section transversale (C-C) étant entre la première section transversale (A-A) et l'extrémité proximale (17) de l'haptique (14), au niveau de la deuxième section transversale (C-C) l'haptique (14) étant plus espacée de la partie optique qu'au niveau de la première section transversale (A-A) pour former une voie d'accès (23) au niveau de la deuxième section transversale (C-C), **caractérisée en ce que**
dans la première section transversale (A-A), l'haptique (14) possède une partie radialement interne (40) avec une première épaisseur (41) mesurée dans une direction radiale par rapport à l'axe optique (OA), et dans la deuxième section transversale (C-C), l'haptique (14) possède une partie radialement interne (40) avec une deuxième épaisseur mesurée dans une direction radiale par rapport à l'axe optique (OA), la deuxième épaisseur étant inférieure à la première épaisseur, et
au niveau de la première section transversale (A-A), l'haptique (14) étant directement adjacente à la périphérie de la partie optique (12).

2. Lentille intraoculaire accommodative de la revendication 1, dans laquelle la première section transversale (A-A) présente une surface externe avec une configuration généralement ovale.

3. Lentille intraoculaire accommodative de la revendication 1, dans laquelle la deuxième section transversale (C-C) présente une surface externe avec une configuration généralement en D.

4. Lentille intraoculaire accommodative de la revendication 1, dans laquelle dans la première section transversale (A-A), la chambre de fluide haptique (22) a une première configuration de chambre de fluide, et dans la deuxième section transversale (C-C), la chambre de fluide haptique (22) a une deuxième configuration de chambre de fluide qui est sensiblement identique à la première configuration de chambre de fluide.

5. Lentille intraoculaire accommodative de la revendication 4, dans laquelle la première configuration de chambre de fluide et la deuxième configuration de chambre de fluide présentent une surface radialement interne (43) qui est plus linéaire qu'une surface radialement externe.

6. Lentille intraoculaire accommodative de la revendication 4, dans laquelle la première configuration de chambre de fluide et la deuxième configuration de chambre de fluide sont sensiblement en D.

7. Lentille intraoculaire accommodative de la revendication 1, dans laquelle dans la première section transversale (A-A), la partie radialement interne (40) est au moins deux fois plus épaisse que la partie radialement externe (42).
